# EUROPEAN PATENT SPECIFICATION

(11) **EP 0 387 757 B1**
(45) Date of publication and mention of the grant of the patent: **15.12.1993**
(21) Application number: 90104641.7
(22) Date of filing: 12.03.1990
(51) Int. Cl.: A61K 7/06, A61K 31/70

(54) **Use of 5'-deoxy-5'methylthioadenosine, S-adenosylmethionine and their salts in the preparation of pharmaceutical compositions favouring hair growth in subjects suffering from baldness**
Verwendung von 5'-Deoxy-5'-methylthioadenosin, s-Adenylmethionin und deren Salzen zur Herstellung pharmazeutischer Präparate zur Förderung des Haarwuchses bei Patienten mit Kahlköpfigkeit
Utilisation des 5'-déoxy-5'-méthylthioadénosine, s-adénosylméthionine et leurs sels pour la préparation de compositions pharmaceutiques favorisant la repousse des cheveux chez des sujets atteints de calvitie

(30) Priority: 13.03.1989 IT 1974789
(43) Date of publication of application: 19.09.1990
(73) Proprietor: BIORESEARCH S.p.A., I-20060 Liscate (Milano) (IT)
(72) Inventor: Maggioni Moratti, Emanuela, I-24100 Bergamo (IT)
(74) Representative: Gervasi, Gemma, Dr.

(56) References cited:
- EP-A- 0 136 463
- EP-A- 0 136 464
- GB-A- 1 555 991

## Description

This invention relates to the use of 5'-deoxy-5′-methylthio-adenosine (MTA), S-adenosylmethionine (SAMe) and their pharmaceutically acceptable salts in the preparation of pharmaceutical compositions favouring hair growth in subjects suffering from baldness, and to the relative pharmaceutical compositions.

Hair does not perform a vital function in man, however it plays a psychological role of primary importance in that the head of hair represents an aesthetic factor, a social factor and a sexual attraction, and its endangering or indeed its loss produces a series of negative and sometimes debilitating psychological effects both in men and women.

The pilus and the hair follicle form a functionally inseparable unit which during the entire life span undergoes growth cycles in which three consecutive stages can be recognised (Kligman A.M., J. Inv. Derm. 33, 307, 1959. Kligman A.M., Arch. Derm., 83, 175, 1961. Zaun H., Aertzl. Kosmetologie 8, 5, 1978):
- an active growth stage (anagen)
- an intermediate stage (catagen)
- a rest stage (telogen)
The anagen stage, with a variable duration of between 3 and 5 years, is characterised by intense metabolic activity at the bulb level, leading to the formation of keratin and melanin and to the lengthening of the hair.

The catagen stage is a transitional stage lasting some weeks. The metabolic activity slows down and the bulb, previously adjacent to the deep dermis, slightly retracts towards the epidermis surface.

The telogen stage corresponds to a period of follicle quiescence. The hair growth ceases. On termination of this period of metabolic rest, which lasts some months, a new hair begins to develop at the base of the follicle and the hair in the telogen stage is pushed towards the cutaneous surface and falls out.

If the hair cycles were synchronized, there would be a massive fall-out of hair after a growth period, as in animal moulting. In contrast, in man hair fall-out is random, with all growth stages present in the same hair region.

A permanent physiological renewal takes place which ensures a constant hair density, particularly as the anagen stage is much longer than the telogen stage. Most of the hair, to the extent of about 90% in the case of healthy hair, is therefore in the anagen stage. As about 100,000-150,000 hairs are in the active growth stage in the case of a young adult, it is estimated that 10,000-15,000 hairs are destined to fall out each year, with an average of about 35 hairs per day.

Consequently the hair consists of various classes of individual hairs ranging from very fine elements with very short cycles to the thickest having a growth cycle of many years. The differing combination of these groups in varying proportions gives the hair a more or less abundant volume independently of the number of individual hairs present on the scalp.

This physiological renewal is however susceptible to individual variations based on the genetic make-up, the sex, the hormone balance and the season (Barman J.M., Astore I, Pecoraro V: J. Inv. Derm. 44, 233, 1965. Barman J.M., Astore I, Pecoraro V: Adv. Biol. Skin vol IX, 1967. Aron Brunetière R., Binet O., Dompmartin-Pernot D.: Révue de Médecin No. 26-27, 1977).

This permanent renewal process of the head of hair can undergo disturbance, as in the case of androgenetic alopecia (Zaun H.: Aertzl. Kosmetologie 8, 5, 1978. Bicham K.D., Shaw D.A.: IFSCC Hamburg 1972).

Alopecia is characterised by a shortening of the anagen stage; passage to the telogen stage is earlier and the subsequent regrowth results in the formation of increasingly shorter hairs. It is the number of very fine hairs with very short cycles which give hair afflicted with alopecia its poor and sparse appearance. As the telogen stage does not change its duration there is also a relative increase in the proportion of hairs in the telogen stage. It is generally considered that a proportion of hairs in the telogen stage exceeding 20% is the sign of alopecia (Kligman A.M.: J. Inv. Derm. 33, 307, 1959. Aron Brunetière R., Binet O., Dompmartin-Pernot D.: Révue de Médecin No. 26-27, 1977).

Therapeutic uses of the product class comprising MTA and SAMe as antivirals, cytostatics and oncogenetic tissue transformation inhibitors are already known and widely described in the literature, such as in GB patent 1,555,991.

Therapeutic uses of this product class as anti-inflammatories, antipyretics, platelet antiaggregants and sleep inducers are also known, as described in USA patents 4,454,122 and 4,373,097.

EP136463 disclose therapeutical injectable compositions based on stable SAMe salts, and of pH between 5 and 8,5, whereas EP136464 describe gastro resistant and enterosoluble compositions allowing oral administrations of stable SAMe salts. These salts for oral use have considerable antiphlogistic and analgesic activity.

Reference should be made to the aforesaid patents for descriptions of MTA and SAMe preparation processes.

MTA and SAMe scalp activity in favouring hair growth or resisting baldness has however never been described.

Many substances have been used up to the present time in the treatment of baldness and hair fall-out, but none of these has given satisfactory results.

For example, in the case of the currently most widely used drug for treating alopecia, ie minoxidil, numerous side effects have been reported, the most frequent of which is allergic contact dermatitis ascribable to the active principle rather than its vehicle (Fielder-Weiss V.C., West D.P., Buys C.M., Rumsfield J.A.: Topical minoxidil dose-response effect in alopecia areata, Arch. Dermatol., 1986, 122, 180-2. Tosti A.: Topical minoxidil useful in 18% of patients with androgenetic alopecia: a study of 430 cases; Dermatologica 1986, 173, 136-8. De Greef H., Hendrickx I., Dooms Goossens A.: Allergic contact dermatitis to minoxidil, Contact Dermatitis 1985, 13, 194-5. Tosti A., Bardazzi F., De Padova M.P., Caponeri G.M., Melino M., Veronesi S.: Contact dermatitis to minoxidil, Contact Dermatitis, 1985, 13, 275-6. Kreindler T.G.: Topical minoxidil in early androgenetic alopecia, J. Am. Acad. Dermatol. 1987, 16, 718-24).

Local symptoms such as irritation and soreness have been occasionally reported. Blood pressure reduction in subjects of normal blood pressure during topical treatment with minoxidil have been encountered by Ranchoff and Bergfeld (Topical minoxidil reduces blood pressure. J. Am. Acad. Dermatol. 1985, 12, 586-7).

The object of the present invention is to describe and claim pharmaceutical compositions which substantially improve the trophicity of the scalp and appendages, and more particularly which favour hair growth in subjects suffering from baldness and in particular with androgenetic alopecia, without side effects.

These objects are attained by using MTA, SAMe and their pharmaceutically acceptable salts in the preparation of pharmaceutical compositions which favour hair growth in subjects suffering from baldness and in particular with androgenetic alopecia.

In this respect, we have surprisingly found that pharmaceutical compositions containing MTA, SAMe and their salts of the present invention are active in favouring hair growth in subjects suffering from baldness and in particular in subjects suffering from androgenetic alopecia.

This surprising pharmacological activity, in no way predictable on the basis of the known pharmacological activity for these products, has been verified by numerous clinical trials. The results obtained in some of these clinical trials are reported hereinafter in order to illustrate the objects and advantages of the present invention.

### Clinical trial 1

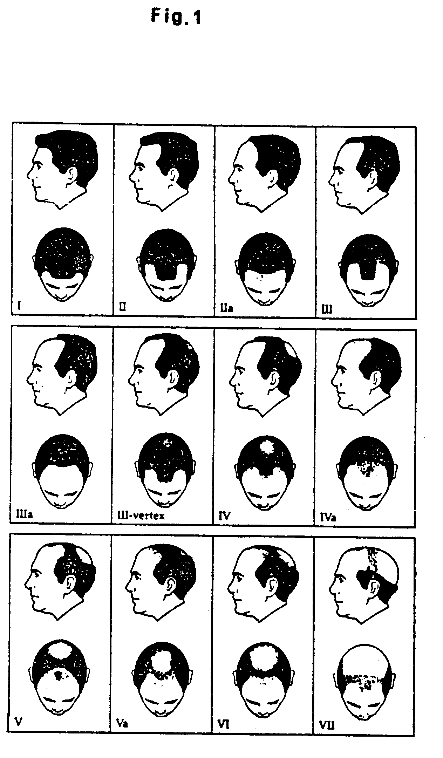

80 subjects of age between 23 and 32 years (average age 27.3) suffering from androgenetic alopecia of grade III vertex and grade IV on the Hamilton scale (Hamilton J.B.: Am. NY Acad. Sci. 1951, 53, 708-28) took part in this clinical trial. The Hamilton scale for classifying baldness is shown in Figure 1.

None of the subjects studied was suffering from any other local or systemic pathology, nor had been treated with other drugs during the two months prior to the clinical trial.

The patients were divided into two numerically equal groups and treated for 4 months either with MTA orally administered at a dose of 600 mg/day or with a placebo, on the basis of a randomized double blind scheme.

The response to the treatment was evaluated independently by the doctor and patient in the following manner.

The patient objectively evaluated the response to treatment with MTA or placebo after 4 months, by choosing one of the following options:
I = no growth
II = minimal growth
III = moderate growth
IV = dense growth
The experimentors objectively evaluated the response to treatment with MTA or placebo after 4 months on the basis of the following parameters:
I = no growth
II = "vellus" hair growth
III = minimal growth of terminal hairs
IV = moderate growth of terminal hairs
V = dense growth of terminal hairs.

Figure 2 shows graphically the subjective evaluation by the patients relative to hair growth after 4 months of therapy with MTA administered orally or with placebo (p<0.05), in terms of the percentage distribution of the 4 hair growth judgement choices as defined above.

Figure 3 shows graphically the objective evaluation by the experimentors relative to hair growth after 4 months of therapy with MTA administered orally or with placebo (p<0.05), in terms of the percentage distribution of the 5 hair growth judgement choices as defined above.

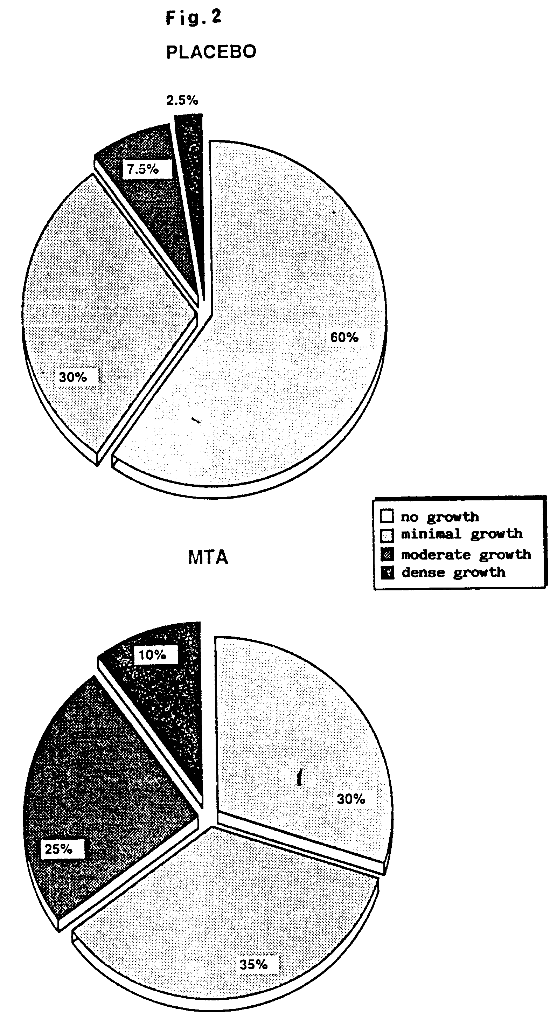

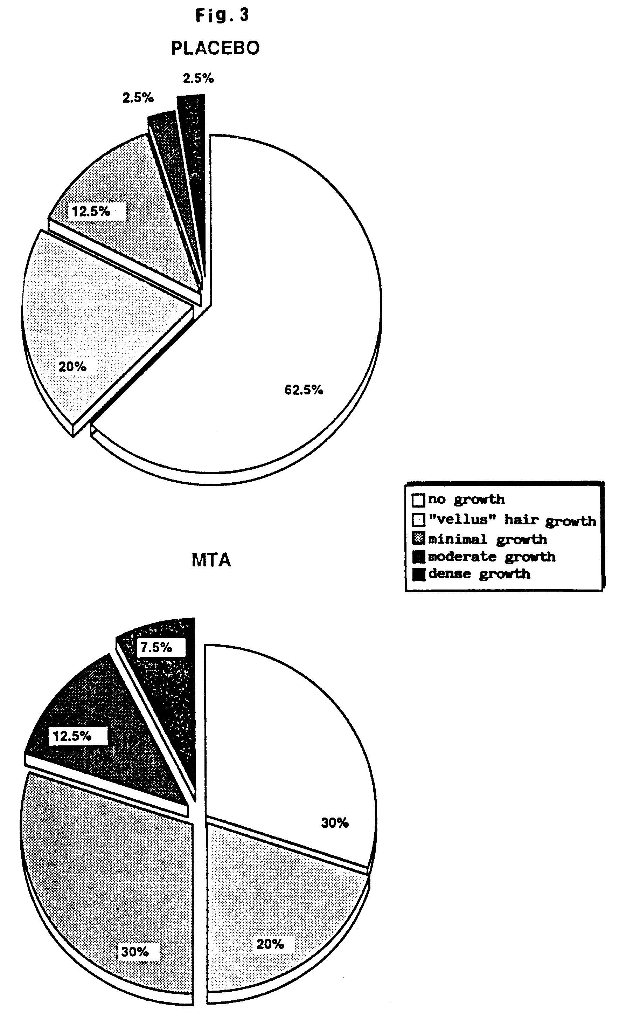

Figures 2 and 3 show that there is a significant difference in the percentage distribution of the 4 judgement choices available to patients or the 5 judgement choices available to experimentors (chi-square test) between the two groups, ie those treated with MTA and those treated with placebo. In particular, there is a significantly greater frequency of type IV or V judgement given by the experimentors in the group treated with MTA than in the group receiving the placebo.

Similar trials were conducted using pharmaceutical compositions of the present invention containing different doses of MTA or SAMe: 50 mg MTA (10 patients), 100 mg MTA (10 patients), 200 mg MTA (10 patients), 400 mg MTA (10 patients), 800 mg MTA (10 patients), 1200 mg MTA (10 patients), 1600 mg MTA (10 patients), 50 mg SAMe (10 patients), 100 mg SAMe (10 patients), 200 mg SAMe (10 patients), 400 mg SAMe (10 patients), 800 mg SAMe (10 patients), 1200 mg SAMe (10 patients), 1600 mg SAMe (10 patients), it being found that these doses also produce significant effects on the parameters involved in evaluating the hair growth.

### Clinical trial 2

120 subjects of age between 25 and 34 years (average age 28.7) suffering from alopecia of grade III vertex and grade IV on the Hamilton scale (see Figure 1) took part in this clinical trial. None of the subjects studied was suffering from any other local or systemic pathology, nor had been treated with other drugs for at least two months prior to the clinical trial. During the treatment period the patients were allowed to wash their hair once a week with a delicate shampoo identical for all. The subjects were divided into two numerically equal groups and treated for 4 months either with 0.25-1% MTA lotion applied topically twice every day, or with a placebo, on the basis of a randomized double blind scheme.

The hair growth was evaluated independently by the doctor and patient after 4 months of treatment in the manner described for clinical trial 1.

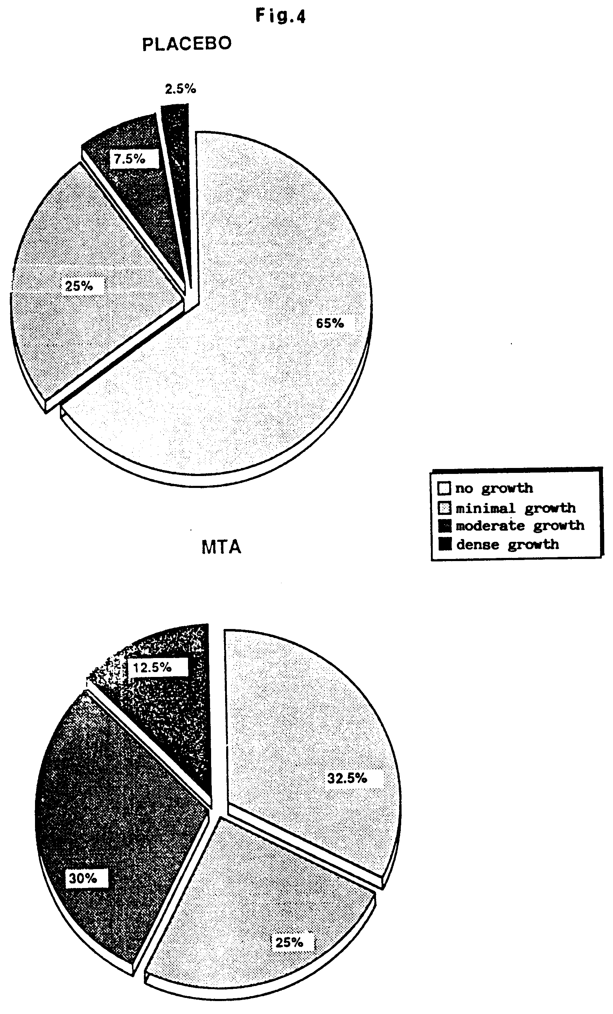

Figure 4 shows graphically the subjective evaluation by the patients relative to hair growth after 4 months of therapy with MTA lotion or placebo, in terms of the percentage distribution of the 4 hair growth judgement choices as defined for clinical trial 1.

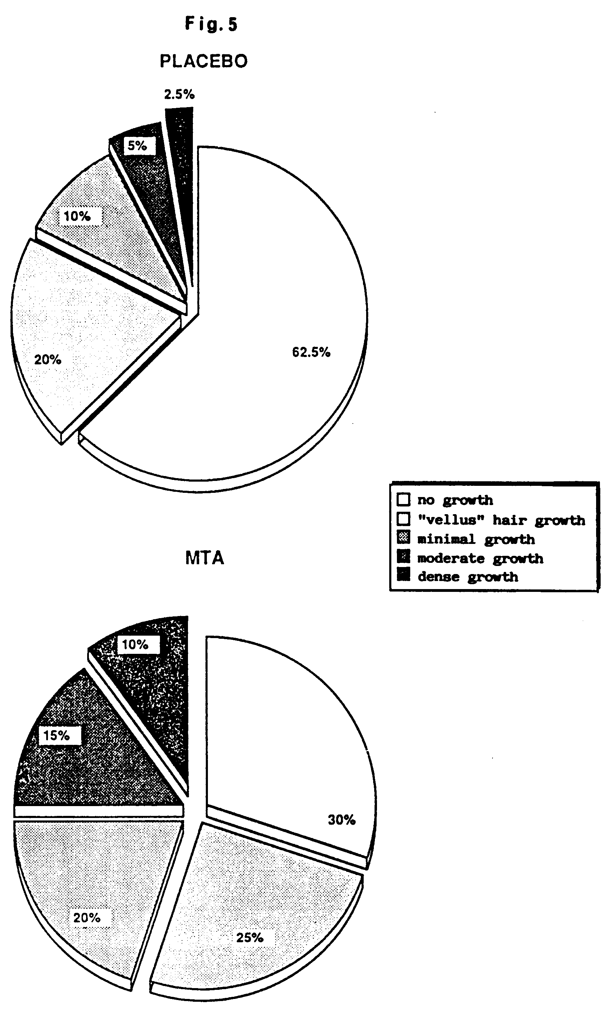

Figure 5 shows graphically the objective evaluation by the experimentors relative to hair growth after 4 months of therapy with MTA lotion or placebo in terms of the percentage distribution of the 5 hair growth judgement choices (p<0.05) as defined for clinical trial 1.

From Figure 4 and even more so from Figure 5 it can be seen that the patients treated topically with MTA lotion showed a significant increase in hair growth compared with the group of subjects receiving the placebo (chi-square test).

Similar clinical trials were conducted using topical pharmaceutical compositions of the present invention containing different doses (all expressed in weight % of solution) of MTA or SAMe: MTA lotion 0.1% (10 patients), MTA lotion 1% (10 patients), MTA lotion 2% (10 patients), MTA lotion 3% (10 patients), MTA lotion 4% (10 patients), MTA lotion 5% (10 patients), SAMe lotion 0.4% (10 patients), SAMe lotion 2% (10 patients), SAMe lotion 4% (10 patients), SAMe lotion 8% (10 patients), it being found that these doses also produce significant effects on the parameters involved in evaluating the hair growth.

A synergic effect was also noted when the therapeutic treatment is effected when alternately using MTA and SAMe with topical, oral and parenteral administration. For example, a synergic effect is obtained by treating the patient on alternate days with topically administered MTA and parenterally administered SAMe, or treating the patient with topically administered SAMe and parenterally administered MTA.

In conclusion, the pharmaceutical compositions of the present invention, whether suitable for systemic or topical administration, have proved to be able to significantly favour hair growth in subjects suffering from baldness and in particular subjects suffering from androgenetic alopecia. It should also be noted that the pharmaceutical compositions prepared from the active principles of the present invention do not induce any negative side effect and are practically free of toxicity at therapeutic doses for any method of administration. The pharmaceutical compositions prepared from MTA, SAMe and their pharmaceutically acceptable salts according to the present invention and suitable for systemic, oral or parenteral administration, such as tablets, suppositories, injectable compositions or transdermic systems, can contain a quantity of active principle, whether MTA or SAMe, of between 50 and 1600 mg, and preferably of between 600 and 1200 mg, whereas the compositions of the present invention suitable for topical administration can contain a quantity of active principle of, in the case of MTA, between 0.1% and 5% by weight, and preferably between 0.25% and 1%, and in the case of SAMe between 0.2% and 16% by weight, and preferably between 2% and 8%, in association with additives and excipients normally used in pharmacy.

Some examples of pharmaceutical compositions according to the present invention are given below for purposes of non-limiting illustration.

### EXAMPLE 1

### Tablet containing 50 mg of MTA

1 tablet contains:

| | |
|---|---|
| MTA sulphate (equivalent to 50 mg MTA) | 58.2 mg |
| Microcrystalline cellulose | 37.8 mg |
| Pregelatinized starch | 151.2 mg |
| Precipitated silica | 0.3 mg |
| Magnesium stearate | 2.5 mg |

### EXAMPLE 2

### Tablet containing 100 mg of MTA

1 tablet contains:

| | |
|---|---|
| MTA ascorbate (equivalent to 100 mg MTA) | 159.3 mg |
| Microcrystalline cellulose | 160.0 mg |
| Lactose 100 mesh | 78.2 mg |
| Precipitated silica | 0.5 mg |
| Magnesium stearate | 2.0 mg |

### EXAMPLE 3

### Controlled-release tablet containing 200 mg of MTA

### Release time = 2 hours

1 tablet contains:

| | |
|---|---|
| MTA citrate (equivalent to 200 mg MTA) | 329.3 mg |
| Dibasic calcium phosphate dihydrate | 48.2 mg |
| Lactose 100 mesh | 100.0 mg |
| Hydroxypropylmethylcellulose | 20.0 mg |
| Magnesium stearate | 2.5 mg |

### EXAMPLE 4

### Controlled-release tablet containing 400 mg of MTA

- Release time =: 2 hours

1 tablet contains:

| | |
|---|---|
| MTA 1,4-butanedisulphonate (equivalent to 400 mg MTA) | 546.8 mg |
| Microcrystalline cellulose | 150.0 mg |
| Dibasic calcium phosphate dihydrate | 118.2 mg |
| Carboxyvinylpolymer | 25.0 mg |
| Glyceryl behenate | 10.0 mg |

### EXAMPLE 5

### Sachet containing 800 mg of MTA

1 sachet contains:

| | |
|---|---|
| MTA p-toluenesulphonate (equivalent to 800 mg MTA) | 1263.3 mg |
| Fructose | 1167.7 mg |
| Lactose 100 mesh | 500.0 mg |
| Precipitated silica | 6.0 mg |
| Aspartame | 10.0 mg |
| Flavouring | 50.0 mg |
| Hydrogenated vegetable oil | 3.0 mg |

### EXAMPLE 6

### Gastroresistant tablet containing 200 mg of SAMe

1 tablet contains:

| | |
|---|---|
| SAMe sulphate p-toluenesulphonate (equivalent to 200 mg SAMe ion) | 384 mg |
| Mannite | 150 mg |
| Precipitated silica | 10 mg |
| Magnesium stearate | 6 mg |
| Cellulose acetophthalate | 15 mg |
| Diethylphthalate | 5 mg |

### EXAMPLE 7

### Gastroresistant tablet containing 400 mg of SAMe

1 tablet contains:

| | |
|---|---|
| SAMe 1,4-butanedisulphonate (equivalent to 400 mg SAMe ion) | 759.2 mg |
| Precipitated silica | 10.0 mg |
| Microcrystalline cellulose | 125.8 mg |
| Magnesium stearate | 5.0 mg |
| Cellulose acetophthalate | 26.2 mg |
| Diethylphthalate | 8.8 mg |

### EXAMPLE 8

### Injectable form containing 200 mg of SAMe

1 bottle contains:

| | |
|---|---|
| SAMe sulphate p-toluenesulphonate (equivalent to 200 mg SAMe ion) | 384 mg |
| Mannite | 200 mg |
| L-lysine | 300 mg |
| Sodium hydroxide | 9 mg |
| Water for injectable preparations | to make up to 5 ml |

### EXAMPLE 9

### Injectable form containing 400 mg of SAMe

1 bottle contains:

| | |
|---|---|
| SAMe 1,4-butanedisulphonate (equivalent to 400 mg SAMe ion) | 759.2 mg |
| L-lysine | 323.0 mg |
| Sodium hydroxide | 9.6 mg |
| Water for injectable preparations | to make up to 5 ml |

### EXAMPLE 10

### Controlled-release injectable form containing 400 mg of SAMe

1 bottle contains:

| | |
|---|---|
| SAMe 1,4-butanedisulphonate (equivalent to 400 mg SAMe ion) | 759.2 mg |
| Polyethyleneglycol 6000 | 100.0 mg |
| Hydroxyethylcellulose | 10.0 mg |
| L-lysine | 323.0 mg |
| Sodium hydroxide | 9.6 mg |
| Water for injectable preparations | to make up to 5 ml |

### EXAMPLE 11

### Controlled-release injectable form containing 200 mg of MTA

1 bottle contains:

| | |
|---|---|
| MTA | 200 mg |
| Polysorbate 80 | 10 mg |
| Polyethyleneglycol 6000 | 100 mg |
| Water for injectable preparations | to make up to 5 ml |

### EXAMPLE 12

### Controlled-release suppository containing 400 mg of MTA

1 suppository contains:

| | |
|---|---|
| MTA | 400 mg |
| Hydroxypropylmethylcellulose | 70 mg |
| Semisynthetic glycerides | 2530 mg |

### EXAMPLE 13

### Transdermic system containing 600 mg of MTA

1 system contains:

| | |
|---|---|
| MTA 1,4-butanedisulphonate (equivalent to 600 mg MTA) | 820.2 mg |
| Glycerin | 1400.0 mg |
| Polyvinyl alcohol | 350.0 mg |
| Polyvinylpyrrolidone | 175.0 mg |
| Purified water | 1575.0 mg |

### EXAMPLE 14

### Transdermic system containing 600 mg of SAMe

1 system contains:

| | |
|---|---|
| SAMe 1,4-butanedisulphonate (equivalent to 600 mg SAMe ion) | 1138.8 mg |
| Fluid silicone | 856.7 mg |
| Precipitated silica | 75.2 mg |

### EXAMPLE 15

### Lotion containing 0.25% of MTA

100 ml contain:

| | |
|---|---|
| MTA sulphate (equivalent to 0.25 g MTA) | 0.29 g |
| Methyl p-hydroxybenzoate | 0.15 g |
| Propyl p-hydroxybenzoate | 0.05 g |
| Purified water to make up to | 100 ml |

### EXAMPLE 16

### Lotion containing 0.5% of MTA

100 ml contain:

| | |
|---|---|
| MTA 1,4-butanedisulphonate (equivalent to 0.5 g MTA) | 0.68 g |
| Methyl p-hydroxybenzoate | 0.15 g |
| Propyl p-hydroxybenzoate | 0.05 g |
| Purified water to make up to | 100 ml |

### EXAMPLE 17

### Lotion containing 1% of MTA

100 ml contain:

| | |
|---|---|
| MTA citrate (equivalent to 1 g MTA) | 1.65 g |
| Methyl p-hydroxybenzoate | 0.15 g |
| Propyl p-hydroxybenzoate | 0.05 g |
| Purified water to make up to | 100 ml |

### EXAMPLE 18

### Spray lotion containing 0.5% of MTA

100 ml contain:

| | |
|---|---|
| MTA 1,4-butanedisulphonate (equivalent to 0.5 g MTA) | 0.68 g |
| Glycerin | 1.00 g |
| Ethyl alcohol | 5.00 g |
| Methyl p-hydroxybenzoate | 0.15 g |
| Propyl p-hydroxybenzoate | 0.05 g |
| Purified water to make up to | 100 ml |

### EXAMPLE 19

### Lotion containing 8% of SAMe

1 bottle contains:

| | |
|---|---|
| SAMe sulphate p-toluenesulphonate (equivalent to 200 mg SAMe ion) | 384.0 mg |
| Methyl p-hydroxybenzoate | 1.6 mg |
| Purified water | 2.5 ml |

### EXAMPLE 20

### Lotion containing 8% of SAMe

1 bottle contains:

| | |
|---|---|
| SAMe 1,4-butanedisulphonate (equivalent to 200 mg SAMe ion) | 379.6 mg |
| Methyl p-hydroxybenzoate | 1.6 mg |
| Purified water | 2.5 ml |

## Claims

1. The use of 5'-deoxy-5'-methylthioadenosine, S-adenosylmethionine and their pharmaceutically acceptable salts in the preparation of pharmaceutical compositions which favour hair growth in subjects suffering from baldness.

2. The use as claimed in claim 1, characterised in that the pharmaceutical compositions are suitable for oral administration.

3. The use as claimed in claim 1, characterised in that the pharmaceutical compositions are suitable for parenteral administration.

4. The use as claimed in claims 2 and 3, characterised in that the quantity of active principle used is between 50 and 1600 mg.

5. The use as claimed in claim 4, characterised in that the quantity of active principle used is preferably between 600 and 1200 mg.

6. The use as claimed in claim 1, characterised in that the pharmaceutical compositions are suitable for topical application.

7. The use as claimed in claim 6, characterised in that the active principle is used in a quantity of between 0.1% and 5% by weight of the solution in the case of 5'-deoxy-5'-methylthio-adenosine and between 0.2% and 16% by weight of the solution in the case of S-adenosylmethionine.

8. The use as claimed in claim 7, characterised in that the active principle is used in a quantity preferably of between 0.25% and 2% by weight of the solution in the case of 5'-deoxy-5'-methylthioadenosine and between 2% and 8% by weight of the solution in the case of S-adenosylmethionine.

## Patentansprüche

1. Verwendung von 5'-Desoxy-5'-methylthioadenosin, S-Adenosylmethionin und pharmazeutisch verträgliche Salzen davon bei der Herstellung von pharmazeutischen Zusammensetzungen, die das Haarwachstum bei an Kahlköpfigkeit leidenden Patienten begünstigen.

2. Verwendung nach Anspruch 1, dadurch gekennzeichnet, daß sich die pharmazeutischen Zusammensetzungen für die orale Verabreichung eignen.

3. Verwendung nach Anspruch 1, dadurch gekennzeichnet, daß sich die pharmazeutischen Zusammensetzungen für die parenterale Verabreichung eignen.

4. Verwendung nach den Ansprüchen 2 und 3, dadurch gekennzeichnet, daß die Wirkstoffmenge 50 - 1600 mg betragt.

5. Verwendung nach Anspruch 4, dadurch gekennzeichnet, daß die Wirkstoffmenge vorzugsweise 600 - 1200 mg beträgt.

6. Verwendung nach Anspruch 1, dadurch gekennzeichnet, daß sich die pharmazeutischen Zusammensetzungen für die topische Anwendung eignen.

7. Verwendung nach Anspruch 6, dadurch gekennzeichnet, daß der Wirkstoff in einer Menge von 0,1 - 5 Gew.-% der Lösung im Fall von 5'-Desoxy-5'-methylthioadenosin und 0,2 - 16 Gew.-% der Lösung im Fall von S-Adenosylmethionin verwendet wird.

8. Verwendung nach Anspruch 7, dadurch gekennzeichnet, daß der Wirkstoff in einer Menge von vorzugsweise 0,25 - 2 Gew.-% der Lösung von Fall von 5'-Desoxy-5'-methylthioadenosin und von 2 - 8 Gew.-% der Lösung im Fall von S-Adenosylmethionin verwendet wird.

## Revendications

1. L'utilisation de la désoxy-5'-méthylthio-5'-adénosine, la S-adénosylméthionine et de leurs sels pharmaceutiquement acceptables pour la préparation de compositions pharmaceutiques favorisant la pousse des cheveux chez des sujets souffrant de calvitie.

2. L'utilisation selon la revendication 1, caractérisée en ce que les compositions pharmaceutiques sont adaptées à l'administration orale.

3. L'utilisation selon la revendication 1, caractérisée en ce que les compositions pharmaceutiques sont adaptées à l'administration parentérale.

4. L'utilisation selon les revendications 2 et 3, caractérisée en ce que la quantité de principe actif utilisée est comprise entre 50 et 1.600 mg.

5. L'utilisation selon la revendication 4, caractérisée en ce que la quantité de principe actif utilisée est de préférence comprise entre 600 et 1.200 mg.

6. L'utilisation selon la revendication 1, caractérisée en ce que les compositions pharmaceutiques sont adaptées à l'application topique.

7. L'utilisation selon la revendication 6, caractérisée en ce que le principe actif est utilisé en une quantité comprise entre 0,1% et 5% en poids de la solution dans le cas de la désoxy-5'-méthylthio-5'-adénosine, et entre 0,2% et 16% en poids de la solution dans le cas de la S-adénosylméthionine.

8. L'utilisation selon la revendication 7, caractérisée en ce que le principe actif est utilisé en une quantité de préférence comprise entre 0,25% et 2% en poids de la solution dans le cas de la désoxy-5'-méthylthio-5'-adénosine, et entre 2% et 8% en poids de la solution dans le cas de la S-adénosylméthionine.
